(19) Europäisches Patentamt / European Patent Office / Office européen des brevets

(11) EP 1 309 536 B1

(12) **EUROPEAN PATENT SPECIFICATION**

(45) Date of publication and mention of the grant of the patent:
**09.06.2010 Bulletin 2010/23**

(21) Application number: **00955767.9**

(22) Date of filing: **18.08.2000**

(51) Int Cl.:
*C07C 51/235* (2006.01) *C07C 51/245* (2006.01)
*C07C 51/285* (2006.01) *C07C 53/126* (2006.01)
*C07C 29/50* (2006.01) *C07C 29/48* (2006.01)
*C07C 45/39* (2006.01) *C07C 45/30* (2006.01)
*C07C 45/29* (2006.01)

(86) International application number:
**PCT/US2000/022865**

(87) International publication number:
**WO 2002/016298 (28.02.2002 Gazette 2002/09)**

(54) **SELECTIVE OXIDATION OF AN ALCOHOL OR KETONE USING A GOLD CATALYST**

SELEKTIVE OXIDATION VON ALKOHOLEN ODER KETONEN MIT EINEM GOLD-KATALYSATOR

OXYDATION SELECTIVE D'ALCOOLS OU DE CETONES À L'AIDE D'UN CATALYSEUR A BASE D'OR

(84) Designated Contracting States:
**DE FR GB NL**

(43) Date of publication of application:
**14.05.2003 Bulletin 2003/20**

(73) Proprietor: **INVISTA Technologies S.à.r.l.**
**8001 Zürich (CH)**

(72) Inventors:
- **HERRON, Norman**
  **Newark, DE 19711 (US)**
- **SCHWARZ, Stephan**
  **Wilmington, DE 19803 (US)**
- **DRULINER, Joe, Douglas**
  **Newark, DE 19711 (US)**

(74) Representative: **Kirsch, Susan Edith**
**Carpmaels & Ransford**
**43-45 Bloomsbury Square**
**London WC1A 2RA (GB)**

(56) References cited:
**EP-A- 0 709 360** **EP-A- 0 965 383**
**US-A- 3 449 413** **US-A- 5 689 000**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### FIELD OF INVENTION

The invention generally relates to an improved catalytic process for selective oxidation utilizing a supported gold catalyst. In particular, the invention generally relates to the oxidation of an alcohol or ketone to produce the corresponding carboxylic acid.

### BACKGROUND

Gold has not seen much use as a catalyst due to its relative unreactivity. Although limited, .several examples of reactions catalyzed by gold have been disclosed. U.S. Patent No. 3,449,413 describes a method for the preparation of carboxylic acid salts using an alkaline aqueous medium and a copper oxide/gold mixture. U.S. Patent Nos. 4,154,762, 4,816,606, and 3,149,166 all use a gold catalyst for the oxidation of a alcohol to form the corresponding ketone, but all perform the reaction at high temperatures and in the gas phase. U.S. Patent No. 5,623,090 oxidized hydrocarbon to form an alcohol or ketone at lower temperatures and in liquid phase, but added hydrogen gas is required. U.S. Patent No. 3,717674 states a gold catalyst may be used for the purification of a terephthalic acid reaction mixture via non-specific oxidation of the by-products therein, but no examples are given.
U.S. Patent No.6,160,183 (Druliner et al.) discloses the use of an improved catalytic process for oxidizing cycloalkanes to form a mixture containing the corresponding alcohol and ketone using a heterogeneous gold catalyst.
The present invention describes methods of utilizing a heterogeneous gold catalyst under mild conditions for the preparation of carboxylic acids and diacids. These compounds are useful as synthetic intermediates and in a variety of industries, such as polymer production.

### SUMMARY OF THE INVENTION

The present invention includes a process for the oxidation of an alcohol or ketone to produce the corresponding carboxylic acid or diacid, comprising contacting the alcohol or ketone with a catalytic amount of a heterogeneous catalyst consisting essentially of gold, wherein the reaction is performed in the liquid phase. Preferably the alcohol or ketone is of the formula

R-OH

or

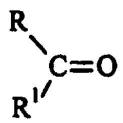

wherein R and $R^1$ are an optionally substituted $C_1$-$C_{20}$ alkyl or aromatic group; most preferred is where the alcohol and ketone are of the formula

R-OH

or

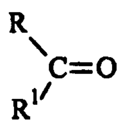

wherein R and $R^1$ are $C_6$-$C_{12}$ alkyl, either linear or cyclic, optionally substituted with alkyl or carboxylic acid groups. Optionally, the heterogeneous catalyst is supported on a catalyst support member. The process can also optionally be run in the presence of added oxidant.

DETAILED DESCRIPTION OF THE INVENTION

The present invention provides a method for the oxidation of an alcohol or ketone to produce the corresponding carboxylic acid in the presence of a catalytic amount of a heterogeneous catalyst consisting essentially of gold.

Advantages of the present heterogeneous catalytic processes, relative to processes employing homogenous metal catalysts, such as metal salts or metal/ligand mixtures, include mild conditions, selectivity to desired products, longer catalyst life, improved yields of useful products, and the absence of soluble metal compounds.

The processes have the advantage over known processes in that they will run in the liquid phase under mild conditions. The conversion rate can vary from little conversion to very high by adjusting the conditions appropriately, as known to anyone skilled in the art. The rate of reaction and selectivity can vary greatly by adjusting the support, oxidant, temperature and time as known to anyone skilled in the art.

The heterogeneous catalysts of the invention include Au, preferably applied to suitable solid supports. The Au is essentially in the form of elemental gold, although some compounds or salts may also be present. The metal to support percentage can vary from 0.01 to 50 percent by weight, and is preferably 0.1 to 10 wt. percent. Suitable, presently preferred supports include $SiO_2$ (silica), $Al_2O_3$ (alumina), C (carbon), $TiO_2$ (titania), MgO (magnesia) or $ZrO_2$ (zirconia). Alumina and titania are particularly preferred supports, and Au supported on alumina is a particularly preferred catalyst of the invention.

Some of the heterogeneous catalysts of the invention can be obtained already prepared from manufacturers, or they can be prepared from suitable starting materials using methods known in the art. These methods can include sol-gel techniques as described in more detail below. Supported gold catalysts can be prepared by any standard procedure known to give well-dispersed gold, such as evaporative techniques or coatings from colloidal dispersions.

In particular, ultra-fine particle sized gold is preferred. Such small particulate gold (often smaller than 10 nm) can be prepared according to Haruta, M., "Size-and Support-Dependency in the Catalysis of Gold", Catalysis Today 36 (1997) 153-166 and Tsubota et al., Preparation of Catalysts V, pp. 695-704 (1991). Such gold preparations produce samples that are purple-pink in color instead of the typical bronze color associated with gold and result in highly dispersed gold catalysts when placed on a suitable support member. These highly dispersed gold particles typically are from 3 nm to 25 nm in diameter.

The catalyst solid support, including $SiO_2$, $Al_2O_3$, carbon, MgO, zirconia, or $TiO_2$, can be amorphous or crystalline, or a mixture of amorphous and crystalline forms. Selection of an optimal average particle size for the catalyst supports will depend upon such process parameters as reactor residence time and desired reactor flow rates. Generally, the average particle size selected will vary from 0.005 mm to 5 mm. Catalysts having a surface area larger than 10 $m^2/g$ are preferred since increased surface area of the catalyst has a direct correlation with increased decomposition rates in batch experiments. Supports having much larger surface areas can also be employed, but inherent brittleness of high-surface area catalysts, and attendant problems in maintaining an acceptable particle size distribution, will establish a practical upper limit upon catalyst support surface area.

The catalysts of the present invention may optionally be silanized by an organosilicon reagent. This treatment prevents fouling of the catalyst, which reduces the catalyst lifetime, by water and organic impurities from the oxidation reactions.

Silanized is defined herein to refer to treatment of the catalyst with either at least one silane, or a mixture of at least one silane and at least one polysiloxane (collectively referred to herein as organosilicon compounds).

Suitable silanes have the formula $R_xSi(R')_{4-x}$ wherein R is a nonhydrolyzable aliphatic, cycloaliphatic or aromatic group having at least 8 to 20 carbon atoms;

R' is a hydrolyzable group such as but not limited to alkoxy, halogen, acyloxy, acetoxy, hydroxy or mixtures thereof; and x=1 to 3.

For example, silanes useful in carrying out the invention include octyltriethoxysilane, nonyltriethoxysilane, decyltriethoxysilane, dodecyltriethoxysilane, tridecyltriethoxysilane, tetradecyltriethoxysilane, pentadecyltriethoxysilane, hexadecyltriethoxysilane, heptadecyltriethoxysilane and octadecyltriethoxysilane. Preferred examples of silanes include R=8-10 carbon atoms; R'=chloro, ethoxy, methoxy, hydroxy or mixtures thereof; and x=1 to 3. Most preferred silanes are R=8 carbon atoms; R'=ethoxy; and x=3. Mixtures of silanes are contemplated equivalents. Weight content of the silane, based on total silanized catalyst is typically about 0.1 to about 3 weight %, preferably about 0.2 to about 2 weight %.

In an alternative embodiment, a mixture of at least one silane with at least one polysiloxane is useful in carrying out the invention. Suitable polysiloxanes have the formula: wherein R is organic or inorganic groups; n=0-3; and m¼2. For example, polydimethylsiloxane (PDMS), vinyl phenylmethyl terminated dimethyl siloxanes, divinylmethyl terminated polydimethyl siloxane and the like are suitable polysiloxanes. PDMS is a preferred polysiloxane. The silane useful in the mixture may be the silane described above with R=8-20 carbon atoms, R'=alkoxy and x=1 preferred. Weight content of the silane and polysiloxane, is 0.1 to 5.0 weight %, preferably from 0.2 to 3 weight %. The ratio of silane to polysiloxane can be 1 silane:2 polysiloxane up to 2 silane: 1 polysiloxane.

The silanes and polysiloxanes are commercially available or can be prepared by processes known in the art

such as those described in "Organosilicon Compounds", S. Pawlenko, et al., New York (1980). The method of addition is not especially critical and the catalyst may be treated with the silane in a number of ways. For example, the silane addition can be made neat or prehydrolyzed to a dry base, from a slurry, a filtration step, during drying or at a size operation such as a fluid energy mill, e.g., micronizer, or media mill as described in greater detail in Niedenzu, et al, U.S. Patent No. 5,501,732, or post blending after micronizing. The polysiloxane addition can be made in conjunction with the silane or post addition to the silanized pigment.

An alternate embodiment that is contemplated is the use of other members of Periodic Groups IV and V in place of Si, such as Ge, P, and As. The catalysts of the invention would thereby be treated with compounds that are the equivalent of the silanes of the instant invention, such as $R_xGe(R')_{4-x}$, $R_xP(R')_{3-x}$, etc.

A "sol-gel technique" is a process wherein a free flowing fluid solution, "sol", is first prepared by dissolving suitable precursor materials such as colloids, alkoxides or metal salts in a solvent. The "sol" is then dosed with a reagent to initiate reactive polymerization of the precursor. A typical example is tetraethoxyorthosilicate (TEOS) dissolved in ethanol. Water, with trace acid or base as catalyst to initiate hydrolysis, is added. As polymerization and crosslinking proceeds, the free flowing "sol" increases in viscosity and can eventually set to a rigid "gel". The "gel" consists of a crosslinked network of the desired material that encapsulates the original solvent within its open porous structure. The "gel" may then be dried, typically by either simple heating in a flow of dry air to produce a xerogel or the entrapped solvent may be removed by displacement with a supercritical fluid such as liquid $CO_2$ to produce an aerogel. These aerogels and xerogels may be optionally calcined at elevated temperatures (>200°C) which results in products which typically have very porous structures and concomitantly high surface areas.

In practice of the invention, the catalysts can be contacted with the reagents by formulation into a catalyst bed, which is arranged to provide intimate contact between catalysts and reactants. Alternatively, catalysts can be slurried with reaction mixtures using techniques known in the art. The processes of the invention are suitable for batch or for continuous processes. These processes can be performed under a wide variety of conditions.

Suitable reaction temperatures for the processes of the invention will vary depending on the particular reaction employed but will generally range from 25°C to 250°C. Temperatures from 50°C to 180°C are typically preferred. Reaction pressures also will vary depending on the particular reaction employed but can preferably range from 69 kPa to 6900 kPa (10-1000 psi) pressure, and pressures from 276 kPa to 4140 kPa (40-600 psi) are more preferred. Reaction time varies in inverse relation to reaction temperature, and typically ranges from 2 to 30 minutes.

The processes can be run with a variety of added oxidants such as but not limited to air, oxygen, hypochlorites such as sodium hypochlorite, peroxides such as hydrogen peroxide, iodosobenzene, and hydroperoxides such as t-butyl hydroperoxide or cyclohexylhydroperoxide.

In the method for the oxidation of an alcohol or ketone to produce the corresponding carboxylic acid, the alcohol and ketone are of the formula R-OH or $RR^1C=O$, wherein R and $R^1$ are an optionally substituted $C_1$-$C_{20}$ alkyl or aromatic group. More preferably, R and $R^1$ are $C_6$-$C_{12}$ alkyl, either linear or cyclic, optionally substituted with alkyl or carboxylic acid groups. Preferred reactions include:

6-hydroxycaproic acid → adipic acid
n-decanol → n-decanoic acid
cyclododecanone → dodecandioic acid
cyclohexanone → hexadioic acid
cyclohexanol → hexanoic acid

This process has the advantage over known processes in that the reaction will run in the liquid phase under mild conditions.

If the starting alcohol or ketone is cyclic, it is possible to make the corresponding diacid:

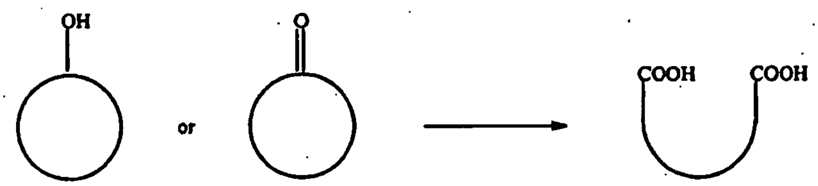

The process is also versatile and can be stopped at any step in the reaction to produce a desired compound, such as but not limited to the alcohol-acid compound:

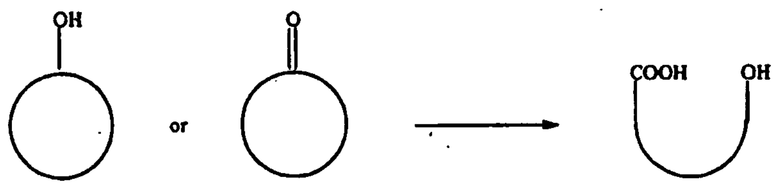
or

Other alkyl aromatics can also be used in the instant process, including but not limited to cumene, toluene, durene, mesitylene, and alkyl-substituted naphthalenes.

The following definitions are used herein and should be referred to for claim interpretation.

| | |
|---|---|
| A | cyclohexanol |
| BSTFA | bis(trimethylsilyl)trifluoroacetamide |
| CB | chlorobenzene |
| CHHP | cyclohexylhydroperoxide |
| HyCap | 6-hydroxycaproic acid, $CH_2OH(CH_2)_4COOH$ |
| K | cyclohexanone |

The following non-limiting Examples are meant to illustrate the invention but are not intended to limit it in any way.

EXAMPLES

GC analyses of reaction products were done either directly on reaction product solutions, or following derivatization using BSTFA (bis(trimethylsilyl)trifluoroacetamide)/1% trimethylchlorosilane, Supelco, Inc., Bellefomte, Pennsylvania), a standard derivatizing agent for GC analyses. GC analyses were done using a 15 m DB-17 capillary column with a 0.32 mm internal diameter (J. & W. Scientific, Folsum, CA).

All calculations of conversion of starting compounds to products are based on molarities of products as determined by GC. The molarity (M) of a given product compound was calculated from the equation:

$$\frac{M_{Compound} = area\%_{Compound} \ X \ M_{CB} \ X \ R.F._{Compound}}{area\%_{CB}}$$

R.F.$_{Compound}$ (GC response factor for a given compound) was determined from calibration solutions containing known amounts of each product compound measured by GC and CB from the equation:

$$\frac{R.F._{Compound} = M_{Compound}/area\%_{Compound}}{M_{CB}/\ area\%_{CB}}$$

In Examples containing no internal GC standard, Conversions were determined from ratios of product compounds/(product compounds + starting compound).

Examples 1 and 3 were done using heterogeneous 1% Au $\alpha$-$Al_2O_3$. These catalysts were prepared by Englehard Corp., 12 Thompson Rd., E. Windsor, CT for E. I. du Pont de Nemours and Company, using a proprietary procedure based on the general procedure shown in Experiment 1 below.

Example 2 was done using heterogeneous 1% Au $\alpha$-$Al_2O_3$ and 1% Au $\alpha$-$TiO_2$. All catalysts were prepared using the general procedures shown below.

EXPERIMENT 1

10 g of titania was slurried into a solution of 0.2 g gold chloride in 25 mL water and 1 drop HCl added. After stirring for 10 mins, the pH was adjusted to 9.6 with sodium carbonate solution (1M). Stirring was continued for 10 more minutes then 0.69g sodium citrate was added and the slurry gently agitated for a further 2 hrs. The gray solid was filtered

off and washed well with water, suction dried and then calcined in flowing air at 250°C for 5 hrs. The recovered purple solid was collected and used for catalysis as described below.

10 g of α-alumina were slurried into a solution of 0.2 g gold chloride in 25 mL water and 1drop HCl added. After stirring for 10 min., the pH was adjusted to 9.6 with sodium carbonate solution (1M). Stirring was continued for 10 more minutes then 0.69 g sodium citrate was added and the slurry gently agitated for a further 2 hrs. The gray solid was filtered off and washed well with water, suction dried and then calcined in flowing air at 250°C for 5 hrs. The recovered purple solid was collected and used for catalysis as described below.

COMPARATIVE EXAMPLE A

Into a ~1 mL GC vial was placed a Teflon®-coated stir bar, 0.0100 g catalyst [10% Co on silica], 0.0206 g (0.156 mmole) HyCap and 0.5 mL $H_2O$. The vial was sealed using a septum cap containing a single hole in it and it was placed in a stainless steel block heater fitted with an o-ring seal and a valve for admitting reaction gases. The block heater was pressurized to 500 psi (3.45 MPa) with air. The contents of the vial were stirred and heated to 160°C (53 minutes heatup time) and held at 160°C for 20 minutes. The reactor was removed from the heating element and was immersed in wet ice. GC analysis of the final reaction product was done on a sample prepared by combining ~30 μl of product solution with ~1.5 mL of BSTFA, and the mixture was stirred at 50°C for 1 hr. The % conversion of HyCap to adipic acid was ~0%.

EXAMPLE 1

Into a ~1 mL GC vial was placed a Teflon®-coated stir bar, 0.0105 g catalyst, 0.0100 g (0.076 mmole) 6-hydroxycaproic acid ( HyCap), 0.0402 g (0.183 m mole) iodosobenzene and 0.5 mL cyclohexane. The vial was sealed using a septum cap and was placed in a heated aluminum block heater. The block heater was pressurized to 500 psi (3.45 MPa) with $N_2$ and the contents of the vial were stirred at 90°C for 10 minutes. The contents of the vial were combined with 1 mL of BSTFA and the mixture was stirred at 50°C for 1 hr. The sample was analyzed by GC. The %conversion of HyCap to adipic acid was -17%.

EXAMPLE 2

5 mL of a stock solution containing 90% p-xylene and 10% acetic acid with 0.1 % o-dichlorobenzene are placed in a shaker tube. 500 mg of catalyst are added and the tube loaded into a rack. The rack was pressured to 500 psi (3.45 MPa) air and heated to 150°C for 5 hrs while shaking vigorously. After cooling the sample was recovered and 5 mL pyridine added. 4-5 Pellets dry 4A molecular sieves were used to dry the sample and then 0.5 mL BSTFA was added to a 1 mL portion of the solution. After stirring for 10 mins the sample was analyzed by GC on a capillary column. Results are shown in Table 1 below.

TABLE 1

| Catalyst | % Conversion | % 4-methylbenzaldehyde | % 4-methylbenzylalcohol | % toluic acid | % 1,4 benzene-dimethanol | % 1,2 di-(4-methylphenyl)-ethane | % terephthalic acid |
|---|---|---|---|---|---|---|---|
| None | 24.5 | 18 | 13 | 66 | 0 | 0.4 | 2.4 |
| 1% Au on $TiO_2$ | 36.2 | 4 | 71 | 9 | 15 | 0.0 | 0.0 |
| 1% Au on $\alpha$-alumina | 9.8 | 6 | 69 | 16 | 9 | 0.0 | 0.0 |

There is a marked shift towards alcohol formation relative to simple autoxidation.

EXAMPLE 3

Into a 1 L GC vial was placed a Teflon®-coated stir bar, 0.0204 g catalyst (1%Au on alpha $Al_2O_3$) 0.0209 g (0.169 mmole) of 5-hydroxymethylfurfural and 0.5 mL methanol. The vial was sealed using a septum cap containing a single hole in it and was placed in stainless steel block heater fitted with an o-ring seal and a valve for admitting reaction gases. The block heater was pressurized to 500 psi (3.45 MPa) with air. The contents of the vial were stirred and heated to 80°C (24 minutes heatup time) and held at 80°C for 2 hr. The reactor was removed from the heating element and was cooled in wet ice. The vial was removed from the reactor, and the methanol removed by purging with a $N_2$ stream at 60°C for about an hour. GC analysis of the final reaction mixture was done on a sample prepared using 1 mL of BSTFA added to the reaction mixture, stirred at 50°C for 1 hr. The ratios of final products was approximately 49% 2-hydroxymethyl-5-carbomethoxyfuran, 37% 2-hydroxymethyl-5-furancarboxylic acid, 2% 2,5-furan dicarboxylic acid and 11% unreacted 5-hydroxymethylfurfural. reaction gases. The block heater was pressurized to 500 psi (3.45 MPa) with air. The contents of the vial were stirred and heated to 160°C (40 minutes heatup time) and held at 160°C for 20 minutes. The reactor was removed from the heating element and was immersed in wet ice. GC analysis of the final reaction product was done on a sample prepared using 1 mL of BSTFA added to the reaction mixture, stirred at 50°C for 1 hr. The %convention of HyCap to adipic acid was 60%. HyCap was also converted to caprolactone (8%) and to a dimer of HyCap (21%). Cyclohexane was converted to K (~1.2%), A (~4.0%), CHHP (~0.12%) and to 2-cyclohexenone (~0.06%).

EXAMPLE 6

Into a ~1 mL GC vial was placed a Teflon®-coated stir bar, 0.0104 g, 0.0111 g (0.084 mmole) HyCap and 0.5 mL $H_2O$. The vial was sealed using a septum cap containing a single hole in it and it was placed in a stainless steel block heater fitted with an o-ring seal and a valve for admitting reaction gases. The block heater was pressurized to 500 psi (3.45 MPa) with air. The contents of the vial were stirred and heated to 160°C (40 minutes heatup time) and held at 160°C for 20 minutes. The reactor was removed from the heating element and was immersed in wet ice. GC analysis of the final reaction product was done on a sample prepared by first evaporating the reaction mixture to dryness at -100°C and with an $N_2$ purge. Next, 1 mL of BSTFA was added to the reaction solids and was stirred at 50°C for 1 hr. The % conversion of HyCap to adipic acid was 97%.

EXAMPLE 7

Into a ~1 mL GC vial was placed a Teflon®-coated stir bar, 0.0100 g catalyst, 0.102 g (0. 0.56 mmole) cyclododecanone and 0.5 g cyclododecane. The vial was sealed using a septum cap containing a single hole in it and it was placed in a stainless steel block heater fitted with an o-ring seal and a valve for admitting reaction gases. The block heater was pressurized to 500 psi (3.45 MPa) with air. The contents of the vial were stirred and heated to 170°C (42 minutes heatup time) and held at 170°C for 30 minutes. The reactor was removed from the heating element and was immersed in wet ice. GC analysis of the final reaction product, treated with BSTFA and stirred at 50°C for 1 hr gave a % conversion of cyclododecanone to dodecandioic acid of -2%.

EXAMPLE 8

Into a -1 mL GC vial was placed a Teflon®-coated stir bar, 0.0103 g catalyst, 0.102 g (0. 0.56 mmole) cyclododecanone and 0.5 g $H_2O$. The vial was sealed using a septum cap containing a single hole in it and it was placed in a stainless steel block heater fitted with an o-ring seal and a valve for admitting reaction gases. The block heater was pressurized to 500 psi (3.45 MPa) with air. The contents of the vial were stirred and heated to 170°C (42 minutes heatup time) and held at 170°C for 30 minutes. The reactor was removed from the heating element and was immersed in wet ice. GC analysis of the final reaction product was done on a sample prepared by first evaporating the reaction mixture to dryness at ~100°C and with an $N_2$ purge. Next, 1 mL of BSTFA was added to the reaction solids and was stirred at 50°C for 1 hr. The %conversion of cyclododecanone to dodecandioic acid was ~6%.

EXAMPLE 9

Into a ~1 mL GC vial was placed a Teflon®-coated stir bar, 0.0101 g catalyst, 0.0210 g (0.159 mmole) HyCap and 0.5 mL $H_2O$. The vial was sealed using a septum cap containing a single hole in it and it was placed in a stainless steel block heater fitted with an o-ring seal and a valve for admitting reaction gases. The block heater was pressurized to 500 psi (3.45 MPa) with air. The contents of the vial were stirred and heated to 160°C (53 minutes heatup time) and

held at 160°C for 20 minutes. The reactor was removed from the heating element and was immersed in wet ice. GC analysis of the final reaction product was done on a sample prepared by combining ~30 μl of product solution with ~1.5 mL of BSTFA, and the mixture was stirred at 50°C for 1 hr. The % conversion of HyCap to adipic acid was 42%.

COMPARATIVE EXAMPLE 9A

Into a ~1 mL GC vial was placed a Teflon®-coated stir bar, 0.0100 g catalyst [10% Co on silica], 0.0206 g (0.156 mmole) HyCap and 0.5 mL $H_2O$. The vial was sealed using a septum cap containing a single hole in it and it was placed in a stainless steel block heater fitted with an o-ring seal and a valve for admitting reaction gases. The block heater was pressurized to 500 psi (3.45 MPa) with air. The contents of the vial were stirred and heated to 160°C (53 minutes heatup time) and held at 160°C for 20 minutes. The reactor was removed from the heating element and was immersed in wet ice. GC analysis of the final reaction product was done on a sample prepared by combining ~30 μl of product solution with ~1.5 mL of BSTFA, and the mixture was stirred at 50°C for 1 hr. The % conversion of HyCap to adipic acid was ~0%.

EXAMPLE 10

Into a ~1 mL GC vial was placed a Teflon®-coated stir bar, 0.0103 g catalyst [1% Au on alpha$Al_2O_3$], supplied by Engelhard, 0.0206 g (0.156 mmole) 6-hydroxycaproic acid (HyCap) and 0.5 mL 0.65 M aqueous NaOCl [Clorox solution]. The vial was sealed using a septum cap and was placed in a heated aluminum block heater. The contents of the vial were stirred at 90°C for 10 minutes. ~30 μL of product solution was combined with 1 mL of BSTFA, and the mixture was stirred at 50°C for 1 hr. The sample was analyzed by GC. The %conversion of HyCap to adipic acid was ~24%.

EXAMPLE 11

Into a ~1 mL GC vial was placed a Teflon®-coated stir bar, 0.0105 g catalyst, 0.0100 g (0.076 mmole) 6-hydroxycaproic acid (HyCap), 0.0402 g (0.183 m mole) iodosobenzene and 0.5 mL cyclohexane. The vial was sealed using a septum cap and was placed in a heated aluminum block heater. The block heater was pressurized to 500 psi (3.45 MPa) with $N_2$ and the contents of the vial were stirred at 90°C for 10 minutes. The contents of the vial were combined with 1 mL of BSTFA and the mixture was stirred at 50°C for 1 hr. The sample was analyzed by GC. The %conversion of HyCap to adipic acid was ~17%.

EXAMPLE 12

5 mL of a stock solution containing 90% p-xylene and 10% acetic acid with 0.1% o-dichlorobenzene are placed in a shaker tube. 500 mg of catalyst are added and the tube loaded into a rack. The rack was pressured to 500 psi (3.45 MPa) air and heated to 150°C for 5 hrs while shaking vigorously. After cooling the sample was recovered and 5 mL pyridine added. 4-5 Pellets dry 4A molecular sieves were used to dry the sample and then 0.5 mL BSTFA was added to a 1 mL portion of the solution. After stirring for 10 mins the sample was analyzed by GC on a capillary column. Results are shown in Table 1 below.

TABLE 1

| Catalyst | % Conversion | % 4-methylbenzaldehyde | % 4-methylbenzylalcohol | % toluic acid | % 1,4 benzene-dimethanol | % 1,2 di-(4-methylphenyl)-ethane | % terephthalic acid |
|---|---|---|---|---|---|---|---|
| None | 24.5 | 18 | 13 | 66 | 0 | 0.4 | 2.4 |
| 1% Au on $TiO_2$ | 36.2 | 4 | 71 | 9 | 15 | 0.0 | 0.0 |
| 1% Au on $\alpha$-alumina | 9.8 | 6 | 69 | 16 | 9 | 0.0 | 0.0 |

There is a marked shift towards alcohol formation relative to simple autoxidation.

EXAMPLE 13

Into a 1 L GC vial was placed a Teflon®-coated stir bar, 0.0204 g catalyst (1%Au on alpha $Al_2O_3$) 0.0209 g (0.169 mmole) of 5-hydroxymethylfurfural and 0.5 mL methanol. The vial was sealed using a septum cap containing a single hole in it and was placed in stainless steel block heater fitted with an o-ring seal and a valve for admitting reaction gases. The block heater was pressurized to 500 psi (3.45 MPa) with air. The contents of the vial were stirred and heated to 80°C (24 minutes heatup time) and held at 80°C for 2 hr. The reactor was removed from the heating element and was cooled in wet ice. The vial was removed from the reactor, and the methanol removed by purging with a $N_2$ stream at 60°C for about an hour. GC analysis of the final reaction mixture was done on a sample prepared using 1 mL of BSTFA added to the reaction mixture, stirred at 50°C for 1 hr. The ratios of final products was approximately 49% 2-hydroxymethyl-5-carbomethoxyfuran, 37% 2-hydroxymethyl-5-furancarboxylic acid, 2% 2,5-furan dicarboxylic acid and 11% unreacted 5-hydroxymethylfurfural.

## Claims

1. A process for the oxidation of an alcohol or ketone to produce the corresponding carboxylic acid or diacid, comprising contacting the alcohol or ketone with a catalytic amount of a heterogeneous catalyst consisting essentially of gold, wherein the reaction is performed in the liquid phase.

2. The process according to Claim 1 wherein the alcohol or ketone is of the formula

   R-OH or

   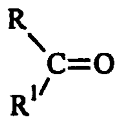

   wherein R and $R^1$ are an optionally substituted $C_1$-$C_{20}$ alkyl or aromatic group, wherein R and $R^1$ can optionally together form a ring.

3. The process according to Claim 1 wherein the heterogeneous catalyst is supported on a catalyst support member.

4. The process according to Claim 3 wherein the catalyst support member is selected from the group consisting of $SiO_2$, $Al_2O_3$, carbon, $TiO_2$, MgO, and zirconia.

5. The process according to Claim 2 wherein the alcohol or ketone are selected from the group consisting of 6-hydroxycaproic acid, n-decanol, and cyclododecanone.

6. The process according to Claim 1 wherein the reaction temperature is from 25°C to 200°C.

7. The process according to Claim 1 wherein the process is run in the presence of added oxidant.

8. The process according to Claim 7 wherein the oxidant is selected from the group consisting of oxygen, iodosobenzene, a hypochlorite and a hydroperoxide.

9. The process according to Claim 3 wherein the gold is present on the support member as well-dispersed particles having a diameter from 3 nm to 25 nm.

## Patentansprüche

1. Verfahren für die Oxidation eines Alkohols oder Ketons zur Bildung der entsprechenden Carbonsäure oder Disäure, umfassend das Kontaktieren des Alkohols oder Ketons mit einer katalytischen Menge eines heterogenen Katalysators bestehend im Wesentlichen aus Gold, wobei die Reaktion in der flüssigen Phase durchgeführt wird.

2. Verfahren nach Anspruch 1, wobei der Alkohol oder das Keton die Formel

R-OH oder

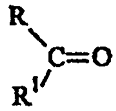

aufweist, wobei R und $R^1$ eine wahlweise substituierte $C_1$-$C_{20}$-Alkyl- oder aromatische Gruppe sind, wobei R und $R^1$ wahlweise zusammen einen Ring bilden können.

3. Verfahren nach Anspruch 1, wobei der heterogene Katalysator auf einem Katalysatorträgerelement getragen wird.

4. Verfahren nach Anspruch 3, wobei das Katalysatorträgerelement aus der Gruppe ausgewählt ist bestehend aus $SiO_2$, $Al_2O_3$, Kohlenstoff, $TiO_2$, MgO und Zirconiumdioxid.

5. Verfahren nach Anspruch 2, wobei der Alkohol oder das Keton aus der Gruppe ausgewählt sind bestehend aus 6-Hyroxycapronsäure, n-Decanol und Cyclododecanon.

6. Verfahren nach Anspruch 1, wobei die Reaktionstemperatur 25 °C bis 200 °C beträgt.

7. Verfahren nach Anspruch 1, wobei das Verfahren in Gegenwart eines hinzugesetzten Oxidationsmittels durchgeführt wird.

8. Verfahren nach Anspruch 7, wobei das Oxidationsmittel aus der Gruppe ausgewählt ist bestehend aus Sauerstoff, Iodosobenzol, einem Hypochlorit und einem Hydroperoxid.

9. Verfahren nach Anspruch 3, wobei das Gold auf dem Trägerelement als gut dispergierte Teilchen vorliegt, die einen Durchmesser von 3 nm bis 25 nm aufweisen.

**Revendications**

1. Procédé d'oxydation d'un alcool ou d'une cétone pour produire l'acide ou le diacide carboxylique correspondant, comprenant la mise en contact de l'alcool ou de la cétone avec une quantité catalytique d'un catalyseur hétérogène essentiellement constitué d'or, dans lequel la réaction est effectuée en phase liquide.

2. Procédé selon la revendication 1, dans lequel l'alcool ou la cétone est de formule

R-OH ou

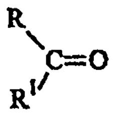

dans laquelle R et $R^1$ sont un groupe alkyle ou aromatique en $C_1$-$C_{20}$ optionnellement substitué,
dans laquelle R et $R^1$ peuvent optionnellement conjointement former un cycle.

3. Procédé selon la revendication 1, dans lequel le catalyseur hétérogène est supporté sur un élément formant support de catalyseur.

4. Procédé selon la revendication 3, dans lequel l'élément formant support de catalyseur est choisi parmi le groupe

constitué de Si02, $Al_2O_3$, du carbone, de $TiO_2$, MgO, et d'oxyde de zirconium.

**5.** Procédé selon la revendication 2, dans lequel l'alcool ou la cétone sont choisis parmi le groupe constitué de l'acide 6-hydroxycaproïque, du n-décanol, et de la cyclododécanone.

**6.** Procédé selon la revendication 1, dans lequel la température réactionnelle est de 25°C à 200°C.

**7.** Procédé selon la revendication 1, dans lequel le procédé est effectué en présence d'un oxydant ajouté.

**8.** Procédé selon la revendication 7, dans lequel l'oxydant est choisi parmi le groupe constitué de l'oxygène, du iodosobenzène, d'une hypochlorite et d'un hydroperoxide.

**9.** Procédé selon la revendication 3, dans lequel l'or est présent sur l'élément formant support sous la forme de particules bien dispersées ayant un diamètre de 3 nm à 25 nm.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- US 3449413 A
- US 4154762 A
- US 4816606 A
- US 3149166 A
- US 5623090 A
- US 3717674 A
- US 6160183 A, Druliner
- US 5501732 A, Niedenzu

### Non-patent literature cited in the description

- **Haruta, M.** Size-and Support-Dependency in the Catalysis of Gold. *Catalysis Today,* 1997, vol. 36, 153-166
- **Tsubota et al.** *Preparation of Catalysts V,* 1991, 695-704
- **S. Pawlenko et al.** *Organosilicon Compounds,* 1980